# EUROPEAN PATENT APPLICATION

(11) **EP 4 039 795 A1**
(43) Date of publication of application: **10.08.2022**
(21) Application number: 20870989.9
(22) Date of filing: 29.09.2020
(51) Int. Cl.: C12N 5/02

(54) **MEDIUM FOR CULTURING HEPATOCYTES, PRODUCTION METHOD FOR HEPATOCYTES, AND HEPATOCYTES**

(30) Priority: 30.09.2019 JP 2019178462
(71) Applicant: FUJIFILM Corporation, Tokyo 106-8620 (JP)
(72) Inventor: MORI, Yusuke, Ashigarakami-gun, Kanagawa 258-8577 (JP); YAMADA, Tadanori, Ashigarakami-gun, Kanagawa 258-8577 (JP)
(74) Representative: Hoffmann Eitle
(86) International application number: PCT/JP2020/036766
(87) International publication number: WO 2021/065849

(57) **Abstract**

An object of the present invention is to provide a medium with which hepatocytes can be cultured while a metabolic function thereof is maintained, a method of producing a hepatocyte using the above medium, and the hepatocyte that is obtained by the above method. According to the present invention, there is provided a medium for culturing a hepatocyte, containing one or more of a ROCK inhibitor, nicotinamide, and an analogous substance of the nicotinamide, in which a content of a serum component is less than 5% by volume, a content of an EGF receptor activating factor is less than 1 ng/mL, and a content of an ALK inhibitor is less than 0.1 µmol/L.

## Description

### Technical Field

The present invention relates to a medium for culturing a hepatocyte. In addition, the present invention relates to a method of producing a hepatocyte using the above medium, and a hepatocyte.

### Background Art

About 95% of drugs are metabolized in the liver, metabolic intermediates are often highly reactive, and toxicity due to the drug metabolites may be problematic. For this reason, it is important to accurately evaluate the risk of liver toxicity. In the related art, the risk of toxicity has been evaluated using experimental animals; however, the predictability of the risk of toxicity in humans has not been sufficient due to the difference in species. For this reason, an in vitro evaluation system using cultured human hepatocytes is used.

By the way, as pluripotent stem cells, an induced pluripotent stem cell (also referred to as an iPS cell), an embryonic stem cell (also referred to as an ES cell), and the like are known. In the field of regenerative medicine, research for the practical application of the iPS cells has been particularly advanced. For example, Patent Document 1 describes that a pluripotent hepatocyte is induced to differentiate into a hepatocyte via a hepatic progenitor cell. As conditions for differentiation of the hepatic progenitor cell, Patent Document 1 describes nicotinamide, Y-27632, EGF, and an ALK inhibitor, which are medium-constituting components. However, Patent Document 1 does not describe culturing while maintaining or enhancing the function of the hepatocyte.

### Prior Art Documents

### Patent Documents

Patent Document 1: WO2017/209211A

### SUMMARY OF THE INVENTION

As described above, the human cultured hepatocyte is used as an in vitro evaluation system for evaluating the risk of liver toxicity. However, it is known that the metabolic activity of the hepatocyte decreases in a short period of time due to culturing in vitro. As a result, in a case where a hepatocyte is cultured using a medium for culturing a hepatocyte, which is currently used, there is a problem that sufficient metabolic activity cannot be obtained for the drug evaluation. Due to such a problem, the evaluations of the toxicity, safety, metabolism, and efficacy of drugs using hepatocytes have not been sufficiently carried out. Accordingly, it is required to establish a method of culturing hepatocytes, with which the activity of hepatocytes in which the drug metabolism can be evaluated is capable of being maintained.

An object of the present invention is to provide a medium with which hepatocytes can be cultured while the metabolic function thereof is maintained, a method of producing a hepatocyte using the above medium, and a hepatocyte that is obtained by the above method.

As a result of diligent studies to achieve the above object, the inventors of the present invention found that in a case where hepatocytes are cultured in a medium which contains one or more of a ROCK inhibitor, nicotinamide, and an analogous substance of the nicotinamide and substantially does not contain a serum component, an EGF receptor activating factor, and an ALK inhibitor, the hepatocytes can be cultured while the metabolic function thereof is maintained. The present invention has been completed based on the above findings.

That is, according to an aspect of the present invention, the following inventions are provided.
<1> A medium for culturing a hepatocyte, comprising:
   one or more of a ROCK inhibitor, nicotinamide, and an analogous substance of the nicotinamide,
   in which a content of a serum component is less than 5% by volume,
   a content of an EGF receptor activating factor is less than 1 ng/mL, and
   a content of an ALK inhibitor is less than 0.1 µmol/L.
<2> The medium according to <1>, in which the medium does not contain the serum component, does not contain the EGF receptor activating factor, and does not contain the ALK inhibitor.
<3> The medium according to <1> or <2>, in which the medium contains the ROCK inhibitor and the nicotinamide or the analogous substance of the nicotinamide.
<4> The medium according to any one of <1> to <3>, in which the ROCK inhibitor is Y-27632.culture medium.
<5> The medium according to any one of <1> to <4>, in which the hepatocyte is a tissue-derived hepatocyte or a pluripotent stem cell-derived hepatocyte.
<6> The medium according to any one of <1> to <5>, in which the medium is a medium for improving a metabolic function or a maintenance period of the hepatocyte.
<7> A method of maintaining and culturing a hepatocyte in the medium according to any one of <1> to <6>.
<8> A method of producing a hepatocyte, comprising culturing the hepatocyte in the medium according to any one of <1> to <6>.
<9> A hepatocyte that is obtained by the method according to <8>.

According to the present invention, hepatocytes can be cultured while the metabolic function thereof is maintained.

### BRIEF DESCRIPTION OF THE DRAWINGS

Figs. 1A to 1C are graphs showing the effects of the respective medium components alone or the combination of two thereof on the CYP3A4 activity per cell.
Fig. 2 is phase-difference images of human iPS cell-derived hepatocytes in a medium (+NAM, ++Y-27632, -EGF, -FBS, and -A83-01) of the embodiment of the present invention.
Fig. 3 is graphs showing expression levels of hepatocyte markers in a case where hepatocytes were cultured using the respective culture solution of human iPS cell-derived hepatocytes.
Fig. 4 is graphs showing expression levels of hepatocyte markers in a case where hepatocytes were cultured using the respective culture solution of human primary hepatocytes.

### DESCRIPTION OF THE PREFERRED EMBODIMENTS

Hereinafter, embodiments for carrying out the present invention will be described in detail.

The abbreviations in the present specification have the following meanings.
AAT: α-1 antitrypsin
ALDH2: Aldehyde dehydrogenase 2
ASGR1: Asialoglycoprotein receptor 1
ALK: Anaplastic lymphoma kinase
CPS1: Carbamoyl phosphate synthetase 1
CYP: Cytochrome P450
DMEM: Dulbecco's modified eagle medium
DMEM/F12 (DMEM Ham's F-12): Dulbecco's modified eagle medium/Nutrition mixture F-12 Ham's
DNmt3L: DNA methyltransferase 3-like
ECAT: ES cell associated transcripts
EGF: Epidermal growth factor
ERAs: ES cell expressed Ras
ESG: Embryonal stem cell-specific gene
Fbx15: F-box protein 15
bFGF: basic Fibroblast growth factor
Fthl17: Ferritin heavy polypeptide-like 17
GAPDH: glyceraldehyde-3-phosphate dehydrogenase
Gdf3: Growth differentiation factor-3
GSTA1: Glutathione S-transferase A1
Grb2: Growth factor receptor-bound protein 2
HGF: Hepatocyte growth factor
Klf: Kruppel-like factor
HNF4a: Hepatocyte nuclear factor 4 alpha
Nr5a1: Nuclear receptor subfamily 5, group A, member 1
Nr5a2: Nuclear receptor subfamily 5, group A, member 2
Oct: Octamer-binding transcription factor
PCR: Polymerase chain reaction
PEPCK: Phosphoenolpyruvate carboxykinase
TDO: Tryptophan 2,3-dioxygenase
UGT1A1: Uridine diphosphate glucuronosyltransferase 1A1
Prdm14: PR/SET domain family 14
Rex1: Reduced-expression 1
ROCK: Rho-associated coiled-coil forming kinase
Sall4: Sal-like protein 4
Sox: Sex determining region Y (SRY)-box
Stat3: Signal transducer and activator of transcription 3
Tcl1: T-cell leukemia/lymphoma 1A
Tert: Telomerase reverse transcriptase
TGF-α: Transforming growth factor-a
UTF1: Undifferentiated embryonic cell transcription factor 1

### [Medium]

The medium of the embodiment of the present invention is a medium for culturing a hepatocyte, including,
(1) one or more of a ROCK inhibitor, nicotinamide, and an analogous substance of the nicotinamide,
(2) in which a content of a serum component is less than 5% by volume,
(3) a content of an EGF receptor activating factor is less than 1 ng/mL, and
(4) a content of an ALK inhibitor is less than 0.1 µmol/L.

As a result of culturing iPS cell-derived hepatocytes and living body-derived primary hepatocytes in the medium of the embodiment of the present invention as described in Examples described later, it was demonstrated that the expression of various metabolic enzymes and hepatocyte markers are increased twice or more as compared with typical culture conditions. Furthermore, it was also demonstrated that it is possible to extend the culture period while maintaining the expression of various metabolic enzymes and hepatocyte markers. In a case where the medium of the embodiment of the present invention is used, it is possible to construct a highly sensitive test of metabolites, a long-term drug exposure test, and various liver pathophysiological models related to metabolism, which have been difficult in the related art.

The "hepatocyte" in the present specification is a tissue-derived hepatic parenchymal cell, a cell having characteristics equivalent to those of a cell generated by differentiation of a pluripotent stem cell into a hepatic parenchymal cell, and a cell having characteristics equivalent to those of an established human hepatic parenchymal cell line. Examples of the characteristics include having a metabolic function, having glycogen accumulation, and having marker expression. Examples of the hepatocyte marker expressed include one or more genes selected from the group consisting of AAT, UGT1A1, TDO, GSTA1, CYP (cytochrome P450) 1A2, CYP2B6, CYP2C8, CYP2C9, CYP2C19, CYP2D6, CYP2E1, CYP3A4, CYP3A5, CYP7A1, albumin, HNF4a, CPS1, PEPCK, and ASGR1. The metabolic function can be measured by quantifying the amounts of metabolites of compounds that are substrates of various enzymes using LC-MS or the like. Glycogen accumulation can be measured by staining fixed cells with Periodic Acid Schiff (PAS). In addition, regarding hepatocyte markers, the presence or absence of marker expression can be evaluated by the mRNA expression analysis using quantitative PCR or the cell staining using antibodies against various markers.

Examples of the ROCK inhibitor include the following compounds.
(+)-(R)-trans-4-(1-aminoethyl)-N-(4-pyridyl)cyclohexanecarboxamide dihydrochloride (Y-27632),
1-(5-isoquinolinesulfonyl)piperazine hydrochloride (HA100), 1-(5-isoquinolinesulfonyl)homopiperazine dihydrochloride (Fasudil/HA-1077),
(S)-(+)-2-methyl-4-glycyl-1-(4-methylisoquinolinyl-5-sulfonyl)homopiperidine dihydrochloride (H-1152),
1-(5-isoquinolinesulfonyl)-2-methylpiperazine (H-7), 1-(5-isoquinolinesulfonyl)-3-methylpiperazine (isoH-7),
N-2-(methylamino)ethyl-5-isoquinoline sulfonamide dihydrochloride (H-8),
N-(2-aminoethyl)-5-isoquinoline sulfonamide dihydrochloride (H-9),
(H-89), N-[2-(p-bromocinnamylamino)ethyl]-5-isoquinoline sulfonamide dihydrochloride
N-(2-guanidinoethyl)-5-isoquinoline sulfonamide hydrochloride (HA-1004),
N-[(LS)-2-hydroxy-1-phenylethyl]-N-[4-(4-pyridinyl)phenyl]-urea (AS1892802),
N-[3-[[2-(4-amino-1,2,5-oxadiazole-3-yl)-1-ethyl-1H-imidazo[4,5-c]pyridine-6-yl]ox y]phenyl]-4-[2-(4-morpholinyl)ethoxy]benzamide (GSK269962),
N-(6-fluoro-1H-indazole-5-yl)-2-methyl-6-oxo-4-(4-(trifluoromethyl)phenyl)-1,4,5,6-tetrahydropyridine-3-carboxamide (GSK429286),
Hydroxyfasudil (HA1100),
2-fluoro-N-[[4-(1H-pyrrolo[2,3-b]pyridine-4-yl)phenyl]methyl]benzenemethanamine (OXA06),
N-[(3-hydroxyphenyl)methyl]-N'-[4-(4-pyridinyl)-2-thiazolyl]urea (RKI1447),
4-(7-{[(3S)-3-amino-1-pyrrolidinyl]carbonyl}-1-ethyl-1H imidazole [4,5-c]pyridine-2-yl)-1,2,5-oxadiazole-3-amine (SB772077B),
N-[2-[2-(dimethylamino)ethoxy]-4-(1H-pyrazole-4-yl)phenyl-2,3-dihydro-1,4-benzod ioxine-2-carboxamide dihydrochloride (SR3677), and
6-chloro-N4-[3,5-difluoro-4-[(3-methyl-1H-pyrrolo[2,3-b]pyridine-4-yl)oxy]phenyl]-2,4-pyrimidinediamine (TC-S7001).

Most preferably, the ROCK inhibitor is Y-27632.

The content of the ROCK inhibitor is preferably 0.001 to 1,000 µmol/L, more preferably 0.01 to 100 µmol/L, and most preferably 0.1 to 10 µmol/L.

Examples of the nicotinamide or nicotinamide analogous substance as the nicotinamide analogous substance of the nicotinamide include nicotinic acid, picoline amide, and 5-methylnicotinamide.

The content of nicotinamide or analogous substance of the nicotinamide is preferably 10 µmol/L to 1 mol/L, more preferably 100 µmol/L to 100 mmol/L, and most preferably 1 mmol/L to 20 mmol/L.

Preferably, the medium of the embodiment of the present invention includes both a ROCK inhibitor and nicotinamide or an analog substance of the nicotinamide.

The content of the serum component is less than 5% by volume, preferably less than 3% by volume, more preferably less than 1% by volume, and still more preferably less than 0.1% by volume. Most preferably, the medium of the embodiment of the present invention does not contain a serum component.

The serum component means a serum component that is used by being added to a cell culture medium, and examples thereof include FBS.

The content of the EGF receptor activating factor is less than 1 ng/mL, preferably less than 0.5 ng/mL, and more preferably less than 0.1 ng/mL. Most preferably, the medium of the embodiment of the present invention does not contain an EGF receptor activating factor.

The EGF receptor activating factor means a factor capable of binding to the EGF receptor and activating the EGF receptor, and examples thereof include EGF which is a ligand of the EGF receptor, TGF-α, amphiregulin, and betacellulin.

The content of the ALK inhibitor is less than 0.1 µmol/L, preferably less than 0.05 µmol/L, and more preferably less than 0.01 µmol/L. Most preferably, the medium of the embodiment of the present invention does not contain an ALK inhibitor.

Examples of the ALK inhibitor include inhibitors of ALK4, ALK5, and ALK7, and specific examples thereof include A83-01 (3-(6-methyl-2-pyridinyl)-N-phenyl-4-(4-quinolinyl)-1H-pyrazole-1-carbothioamide: CAS number: 909910-43-6). Examples of other ALK inhibitors include SB431542 (4-[4-(1,3-benzodioxole-5-yl)-5-(2-pyridinyl)-1H-imidazole-2-yl]benzamide: CAS number: 301836-41-9).

The medium of the embodiment of the present invention can be produced using a basal medium as long as the above requirements (1) to (4) are satisfied. Examples of the basal medium include a Dulbecco's modified eagle medium (DMEM), a mixed medium of DMEM and F12 (DMEM:F12 = 1:1), an RPMI medium (a GIBCO (registered trade name) RPMI 1640 medium, or the like), a mixed medium of DMEM/F12 and RPMI (DMEM/F12:RPMI = 1:1), and Knockout^{™} D-MEM (Thermo Fisher Scientific, Inc.).

In addition to the above basal medium, a medium may be obtained by adding additive components such as a serum substitute (for example, a B-27 supplement, an N-2 supplement, or the like), non-essential amino acids (MEM non-essential amino acids solution, or the like), L-glutamine, L-ascorbic acid, an antibiotic (penicillin, streptomycin, gentamicin, or the like), an iCell hepatocytes 2.0 medium supplement (FUJIFILM Wako Pure Chemical Corporation), insulin, transferrin, selenium, N-acetyl-cysteine, HGF, dexamethasone, and oncostatin M in any combination (preferably, in a combination of all of the above-described additive components).

As the hepatocyte, a tissue-derived hepatocyte or a pluripotent stem cell-derived hepatocyte can be preferably used.

The tissue-derived hepatocyte may be any hepatocyte collected from a living body, may be a primary hepatocyte, or may be an established hepatocyte line; however, it is preferably a primary hepatocyte. A human primary hepatocyte is particularly preferable. As the human primary hepatocyte, a commercially available product such as Thermo Fisher Scientific Inc., Cat #: HMCPP5 may be used.

Examples of the pluripotent stem cell include an embryonic stem cell (an ES cell), an embryonic germ cell (an EG cell), and an induced pluripotent stem cell (an iPS cell); however, the pluripotent stem cell is not limited thereto as long as it has differentiation pluripotency and proliferation ability (self-renewal ability). It is preferable to use an ES cell or an iPS cell, and it is more preferable to use an iPS cell.

The "induced pluripotent stem cell (the iPS cell)" is a cell that has differentiation pluripotency (multiple differentiation potency) and proliferation ability and that is prpared by reprogramming a somatic cell by introducing reprogramming factors or the like. The somatic cell that is used in the preparation of the iPS cell is not particularly limited, and any somatic cell can be used. For example, a human adult-derived somatic cell (that is, a mature somatic cell) may be used in addition to the fetal somatic cell. Examples of the somatic cell include: (1) tissue stem cells (somatic stem cells) such as a neural stem cell, a hematopoietic stem cell, a mesenchymal stem cell, and a dental pulp stem cell; (2) tissue progenitor cells; and (3) differentiated cells such as a fibroblast (a skin cell and the like), an epithelial cell, a hepatocyte, a lymphocyte (T cell or B cell), an endothelial cell, a muscle cell, a hair cell, a gastric mucosal cell, an intestinal cell, a splenocyte, a pancreatic cell (an exocrine pancreatic cell and the like), a brain cell, a lung cell, a kidney cell, and a skin cell. The living body from which the somatic cell is derived is not particularly limited, and examples thereof include, a human and a non-human animal (for example, a monkey, a sheep, a cow, a horse, a dog, a cat, a rabbit, a rat, and a mouse). The living body is preferably a human.

The reprogramming factor to be introduced into a somatic cell is not particularly limited. Examples thereof include Oct3/4, Klf4, c-Myc, Sox2, Nanog, Klf2, L-Myc, N-Myc, Klf5, Lin28, Tert, Fbx15, ERas, ECAT15-1, ECAT15-2, Tcl1, β-catenin, ECAT1, Esg1, Dnmt3L, ECAT8, Gdf3, Sox15, Fthl17, Sall4, Rex1, UTF1, Stella, Stat3, Grb2, Prdm14, Nr5a1, Nr5a2, and E-cadherin. Here, two or more genes can be randomly selected from these gene groups and introduced in any combination. Among these, a combination having at least Oct3/4, Sox2, Klf4, and c-Myc, a combination having at least Oct3/4, Sox2, Klf4, and L-Myc, or a combination having at least Oct3/4, Sox2, Nanog, and Lin28 preferable.

In addition, a gene to be introduced and a cell to which the gene is introduced are preferably derived from the same species. For example, a gene to be introduced into a human-derived cell is preferably a human gene. For example, a combination having at least human OCT3/4, human SOX2, human KLF4, and human MYC, a combination having at least OCT3/4, SOX2, KLF4, and L-MYC, or a combination having at least hunan OCT3/4, human SOX2, human NANOG, and human LIN28 is preferable.

The gene as the reprogramming factor can be introduced into a somatic cell using a gene expression vector. The gene expression vector is not particularly limited, and examples thereof include a virus vector, a plasmid vector, an artificial chromosome vector, and a transposon vector. Examples of the virus vector include a retrovirus vector, an adenovirus vector, a Sendai virus vector, a lentivirus vector, and an adeno-associated virus vector.

The iPS cell may be prepared in-house by introducing reprogramming factors into a somatic cell. Alternatively, a cell provided or sold by a research institution or a company may be obtained.

For example, 201B7, 253G1, 253G4, 1201C1, 1205D1, 1210B2, 1231A3, 1383D2, 1383D6, iPS-TIG120-3f7, iPS-TIG120-4f1, iPS-TIG114-4f1, CiRA086Ai-m1, CiRA188Ai-M1, or iRA188Ai-W1 provided by Kyoto University iPS Cell Research Institute. can be obtained and used.

In addition, it is possible to purchase cells from iPS cell banks, prepared by National Institutes of Health (NIH), California Institute of Regenerative Medicine, New York Stem Cell Foundation, European Bank for induced pluripotent Stem Cells, and the like.

The pluripotent stem cell-derived hepatocyte can be obtained by inducing differentiation of a pluripotent stem cell into the hepatocyte. The method of inducing differentiation of a pluripotent stem cell into the hepatocyte can be carried out according to a method known to those skilled in the art or a method according to the known method. For example, a pluripotent stem cell can be induced to differentiate into the hepatocyte by suspension culture or adhesion culture using a culture medium to which hepatocyte growth factor (HGF) has been added.

As the culture conditions for inducing differentiation into the hepatocyte, the conditions generally adopted in the culture of animal cells can be adopted. That is, the culture can be carried out in an environment of, for example, 37°C and 5% carbon dioxide gas.

The induction of differentiation into the hepatocyte can be confirmed by detecting markers characteristically expressed in the hepatocyte (for example, CYP3A4 involved in drug metabolism, ALDH2 involved in alcohol metabolism, albumin, and the like). The detection of hepatocyte markers can be confirmed by a genetic engineering technique such as reverse transcriptase-polymerase chain reaction (RT-PCR) or real-time quantitative polymerase chain reaction, or by enzyme immunoassay or immunostaining.

As the pluripotent stem cell-derived hepatocyte, a commercially available product may be used. For example, FUJIFILM Wako Pure Chemical Corporation, Cat #: C1026 or the like can be used.

The medium of the embodiment of the present invention can be used as a medium for improving the metabolic function or a maintenance period of the hepatocyte. The medium of the embodiment of the present invention can be used as a medium for evaluation systems of in vitro toxicity, safety, metabolism, and efficacy of drugs using hepatocytes.

Various enzymes and proteins (CYP3A4, CYP1A2, CYP2B6, CYP2E1, CYP2C9, CYP2C19, CYP2D6, UGT, SULT, G6PC, Albumin, ammonia metabolizing enzymes) that are said to be stably and highly expressed in the liver of the living body affects drugs incorporated into the body. The evaluation systems of toxicity, safety, metabolism, and efficacy of drugs with respect to the liver require the expressions of various enzymes and proteins, which are close to those of the living body. The medium of the embodiment of the present invention can be used as a medium for constructing an in vitro evaluation system with improved hepatocyte function, whereby a model for evaluating effects on the liver in drug discovery can be constructed.

### [Method of producing hepatocyte, and hepatocyte]

The present invention provides a method of producing a hepatocyte, which includes culturing the hepatocyte in the medium of the embodiment of the present invention described above. In addition, the present invention provides a hepatocyte that is obtained by the above-described method.

The form of the culture is not particularly limited; however, examples thereof include seeding hepatocytes on a plate (a 96-well plate or the like) coated with collagen, using the medium of the embodiment of the present invention, and culturing them under the conditions of 37°C and 5% carbon dioxide gas.

The culture period is not particularly limited. It may be, for example, 1 hour or more, 2 hours or more, 4 hours or more, 8 hours or more, 12 hours or more, 24 hours or more, 2 days or more, 3 days or more, 4 days or more, and 1 week or more, and it may be 12 weeks or less, 10 weeks or less, 8 weeks or less, 6 weeks or less, 4 weeks or less, 3 weeks or less, or 2 weeks or less.

The hepatocyte according to the embodiment of the present invention can be used for screening medicine candidate compounds for the treatment of various diseases. For example, the toxicity, safety, metabolism, or efficacy of a medicine candidate compound can be evaluated by adding, to hepatocytes, the medicine candidate compound alone or in combination with other drugs and analyzing the metabolite of the medicine candidate compound, or detecting the changes in cell morphology or function, the increase or decrease of various factors, the gene expression profiling, and the like.

Furthermore, the hepatocyte according to the embodiment of the present invention is applicable to the treatment of various liver diseases. In particular, the use as a material for regeneration/reconstruction of a damaged liver tissue (including dysfunction) is considered, and it is expected to contribute to regenerative medicine.

The present invention will be more specifically described using Examples below; however, the present invention is not limited by Examples.

### Examples

### [Example 1]

### [Culture of human iPS cell-derived hepatocyte]

For the cell culture medium, to a medium obtained by mixing equal amounts of a DMEM/F12 basal medium and an RPMI 1640 basal medium, each of the components was added so that the concentration thereof was to be the following final concentration.
1× B-27 supplement (Thermo Fisher Scientific Inc.);
1× N-2 supplement (Thermo Fisher Scientific Inc.);
1× MEM non-essential amino acids solution (Thermo Fisher Scientific Inc.);
1.2 mmol/L L-glutamine (Thermo Fisher Scientific Inc.);
0.5 mmol/L L-ascorbic acid (SIGMA-ALDRICH Co., LLC);
1× penicillin-streptomycin-glutamine (Thermo Fisher Scientific Inc.);
12.5 µg/mL gentamicin (Thermo Fisher Scientific Inc.),
1× iCell hepatocytes 2.0 medium supplement (FUJIFILM Wako Pure Chemical Corporation);
1× insulin, transferrin, selenium, ethanolamine solution (Thermo Fisher Scientific Inc.);
90 µmol/L N-acetyl-cysteine (SIGMA-ALDRICH Co., LLC);
10 ng/mL recombinant human HGF (PeproTech Inc.);
10⁻⁷ mol/L dexamethasone (FUJIFILM Wako Pure Chemical Corporation);
10 ng/mL recombinant human oncostatin M (R&D Systems Inc.)

Further, to the above composition, the following components were added in combination to prepare culture media for various hepatocytes,
nicotinamide (also denoted as NAM) (FUJIFILM Wako Pure Chemical Corporation) (10 mmol/L, 5 mmol/L, 0 mmol/L);
Y-27632 (FUJIFILM Wako Pure Chemical Corporation) (5 µmol/L, 2.5 µmol/L, 0 µmol/L);
fetal bovine serum (FBS) (Thermo Fisher Scientific Inc.) (5%, 0%);
recombinant human EGF (PeproTech Inc.) (5 ng/mL, 0 ng/mL); and
A83-01 (Tocris Bioscience) (2.5 µmol/L, 0 µmol/L).

Using the above-described various media, human iPS cell-derived hepatocytes (FUJIFILM Wako Pure Chemical Corporation, Cat #: C1026) were seeded on a collagen-coated 96-well plate at 1× 10⁵ cells per well and cultured in an incubator at 37°C and 5% carbon dioxide gas, and the medium was exchanged every 24 hours. The luminescence intensity by a P450-Glo CYP3A4 assay (Promega Corporation) was measured by using an EnSpire multimode plate reader (PerkinElmer, Inc.) and corrected by the number of cells in the well to calculate the CYP3A4 activity value per cell in the culture period of 1 week.

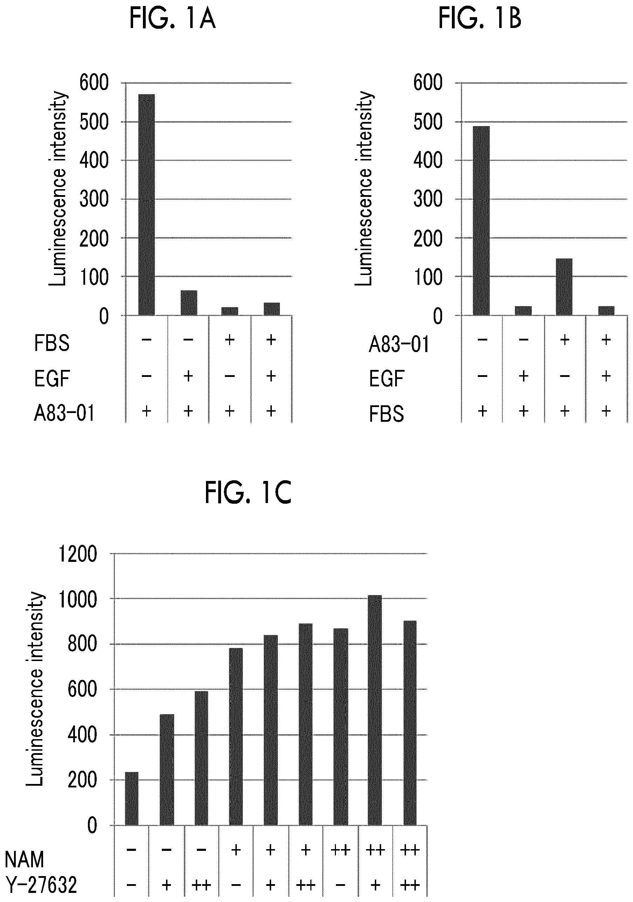

Figs. 1A to 1C shows the results of the measured activity values depending on the presence or absence of each of the factors and the combination of the two factors.

Figs. 1A and 1B show the effects of FBS, EGF, and A83-01 on a medium that contains 5 mmol/L nicotinamide and 2.5 µmol/L Y-27632. Fig. 1C shows the effects of nicotinamide and Y-27632 on a medium that does not contain FBS, EGF, and A83-01. The notation in Fig. 1 is as follows.
FBS
+: 5%
-: 0%
EGF
+: 5 ng/mL
-: 0 ng/mL
A83-01
+: 2.5 µmol/L
-: 0 µmol/L
Nicotinamide
++: 10 mmol/L
+: 5 mmol/L
-: 0 mmol/L
Y-27632
++: 5 µmol/L
+: 2.5 µmol/L
-: 0 µmol/L

From the results of Fig. 1C, it has been found that nicotinamide and Y-27632 are positive factors for improving the function of hepatocytes. Further, from the results of Fig. 1A, it has been found that in hepatocytes cultured in the medium that does not contain FBS and EGF, the CYP3A4 activity is synergistically improved as compared with a case where hepatocytes are cultured in a medium in which FBS and EGF are removed singly, as compared with hepatocytes cultured in the medium that contains FBS and EGF. Similarly, from the results of Fig. 1B, it has been found that in hepatocytes cultured in the medium that does not contain EGF and A83-01, the CYP3A4 activity is synergistically improved as compared with a case where hepatocytes are cultured in a medium in which EGF and A83-01 are removed singly, as compared with hepatocytes cultured in the medium that contains EGF and A83-01.

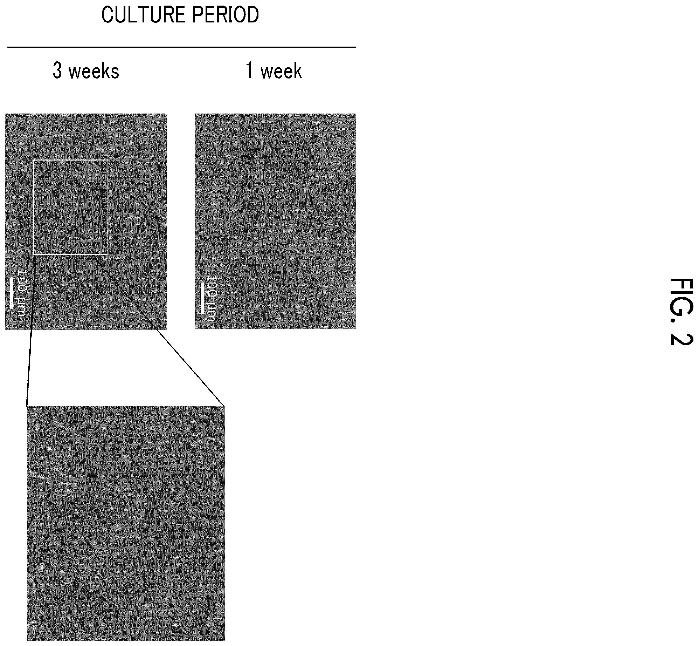

Human iPS cell-derived hepatocytes (FUJIFILM Wako, Cat #: C1026) were seeded on a 96-well plate at 1×10⁵ cells per well and cultured for 1 to 3 weeks in 100 µL of the medium of the embodiment of the present invention, which contains 5 mmol/L nicotinamide, 5 µmol/L Y-27632 and does not contain fetal bovine serum (FBS), recombinant human EGF, and A83-01. The phase difference images of the cells after culture are shown in Fig. 2. Further, human iPS cell-derived hepatocytes (FUJIFILM Wako, Cat #: C1026) seeded at 2.5 × 10⁴ cells per well were cultured under the same culture conditions for 1 to 3 weeks. The cultured cells were detached from the plate with accutase (Innovative Cell Technologies, Inc.), the number of cells was counted using a hemocytometer, and the number of cells after 3 weeks of culture was divided by the number of cells after 1 week of culture to calculate the proliferation rate of the hepatocytes.

From the results shown in Fig. 2, in a case where hepatocytes were cultured in the medium of the embodiment of the present invention, paving stone-like and hepatocyte-like morphology and fine bile duct formation were observed in the culture period of 1 week and 3 weeks. In addition, it was found that the proliferation rate of the hepatocytes became about 1.6 times from 1 week to 3 weeks, and it was seen that in the culture of hepatocytes using the medium of the embodiment of the present invention, the cell proliferation of hepatocytes is maintained for a long period of time.

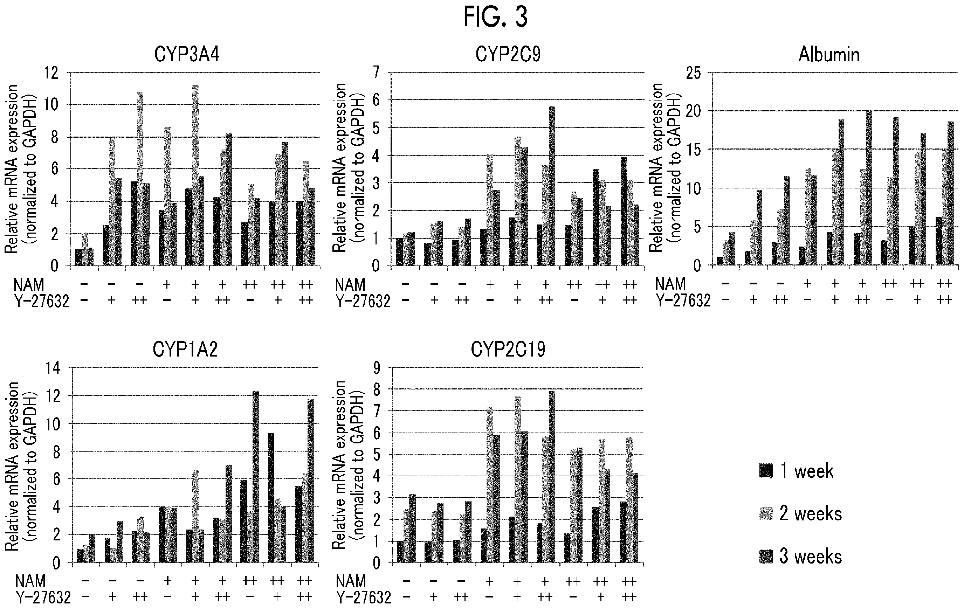

In addition, in a medium obtained by changing the contents of nicotinamide and Y-27632 in the medium that does not contain fetal bovine serum (FBS), recombinant human EGF, and A83-01, the mRNA amounts of various genes (CYP3A4, CYP1A2, CYP2C9, CYP2C19, and Albumin) expressed in the cell during the culture of 1 week, 2 weeks, and 3 weeks were measured using TaqMan (registered trade name) probes (Thermo Fisher Scientific Inc.) by quantitative PCR. After correcting the expression levels of various genes with GAPDH, the relative gene expression levels are shown in Fig. 3 in a case where the gene expression levels after 1 week of culture in the medium that does not contain nicotinamide and Y-27632 is set to 1. Table 1 shows the TaqMan (registered trade name) probes used.

**[Table 1]**

| Gene | Assay ID |
|---|---|
| GAPDH | Hs02786624_g1 |
| CYP3A4 | Hs00604506_m1 |
| CYP1A2 | Hs00167927_m1 |
| CYP2C9 | Hs02383631_s1 |
| CYP2C19 | Hs00426380_m1 |
| Albumin | Hs00609411_m1 |

From the results shown in Fig. 3, it was seen that in the culture periods of 1 week, 2 weeks, and 3 weeks, the expression of hepatocyte markers in iPS cell-derived hepatocytes are improved, and it was suggested that culture can be carried out for a long period of time while the metabolic function of the hepatocyte is maintained in a case where the culture is carried out in the medium of the embodiment of the present invention, which contains at least one of nicotinamide or Y-27632 and does not contain FBS, EGF, and A83-01, as compared with a case of culture carried out with a medium that does not contain nicotinamide, Y-27632, FBS, EGF, and A83-01.

[Example 2]

### [Culture of primary human hepatocyte]

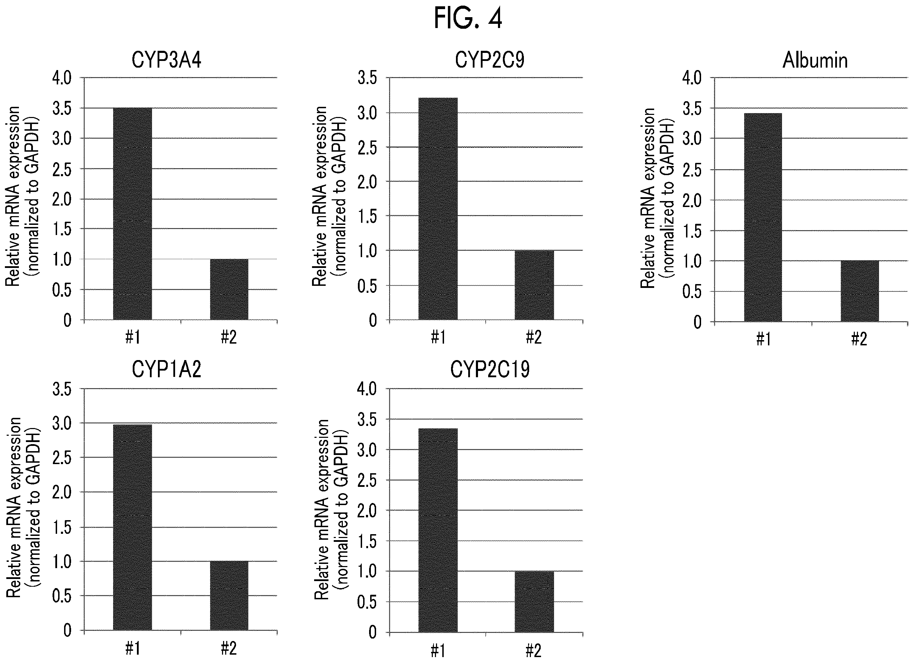

Human primary hepatocytes (Thermo Fisher Scientific Inc., Cat #: HMCPP5) were seeded on a collagen-coated 96-well plate at 1×10⁵ cells per well and cultured in 100 µL of the medium (the medium #1) of the embodiment of the present invention, which contains 5 mmol/L nicotinamide and 5 µmol/L Y-27632 and does not contain fetal bovine serum (FBS), recombinant human EGF, and A83-01, in an incubator at 37°C and 5% carbon dioxide gas, and after 24 hours, the medium was exchanged. For comparison, culture was carried out, under the same culture conditions as the above culture conditions except for the medium compositions, in a medium (a medium #2), which contains 5 mmol/L nicotinamide, 5 µmol/L Y-27632, 5% fetal bovine serum (FBS), 5 ng/mL recombinant human EGF, and 2.5 µmol/L A83-01, and the mRNA amounts of various genes (CYP3A4, CYP1A2, CYP2C9, CYP2C19, and Albumin) expressed in the cell after 48 hours of culture were measured using TaqMan (registered trade name) probes (Thermo Fisher Scientific Inc.) by quantitative PCR in the same manner as in Example 1. Fig. 4 shows the relative gene expression levels in a case where the gene expression levels after 48 hours of culture in the medium #2 are set to 1.

From the results shown in Fig. 4, it was found that from the gene expression evaluation by quantitative PCR 48 hours after the culture, in a case where the medium (the medium #1) of the embodiment of the present invention is used, the expression of the hepatocyte marker is high by about three times as compared with the comparative medium (medium #2).

## Claims

1. A medium for culturing a hepatocyte, comprising:
one or more of a ROCK inhibitor, nicotinamide, and an analogous substance of the nicotinamide,
wherein a content of a serum component is less than 5% by volume,
a content of an EGF receptor activating factor is less than 1 ng/mL, and
a content of an ALK inhibitor is less than 0.1 µmol/L.

2. The medium according to claim 1,
wherein the medium does not contain the serum component, does not contain the EGF receptor activating factor, and does not contain the ALK inhibitor.

3. The medium according to claim 1 or 2,
wherein the medium contains the ROCK inhibitor and the nicotinamide or the analogous substance of the nicotinamide.

4. The medium according to any one of claims 1 to 3,
wherein the ROCK inhibitor is Y-27632.

5. The medium according to any one of claims 1 to 4,
wherein the hepatocyte is a tissue-derived hepatocyte or a pluripotent stem cell-derived hepatocyte.

6. The medium according to any one of claims 1 to 5,
wherein the medium is a medium for improving a metabolic function or a maintenance period of the hepatocyte.

7. A method of maintaining and culturing a hepatocyte in the medium according to any one of claims 1 to 6.

8. A method of producing a hepatocyte, comprising culturing the hepatocyte in the medium according to any one of claims 1 to 6.

9. A hepatocyte that is obtained by the method according to claim 8.
